# EUROPEAN PATENT SPECIFICATION

(11) **EP 2 928 441 B1**
(45) Date of publication and mention of the grant of the patent: **11.07.2018**
(21) Application number: 12806263.5
(22) Date of filing: 06.12.2012
(51) Int. Cl.: A61K 8/27, A61K 8/44, A61Q 11/00, A61K 8/90, A61K 8/19

(54) **SURFACTANT SYSTEMS FOR ZINC CONTAINING COMPOSITIONS**
TENSIDSYSTEME FÜR ZINKHALTIGE ZUSAMMENSETZUNGEN
SYSTÈMES TENSIOACTIFS POUR COMPOSITIONS CONTENANT DU ZINC

(43) Date of publication of application: 14.10.2015
(73) Proprietor: Colgate-Palmolive Company, New York, NY 10022 (US)
(72) Inventor: ROBINSON, Richard Scott, Belle Mead NJ 08502 (US); JOSIAS, Wilbens, North Plainfield New Jersey 07063 (US)
(74) Representative: Wibbelmann, Jobst
(86) International application number: PCT/US2012/068108
(87) International publication number: WO 2014/088575

(56) References cited:
- WO-A1-00/78270
- WO-A1-2008/145475
- WO-A1-2009/099455
- WO-A1-2012/057739

## Description

### BACKGROUND

There is a need in the art for oral care compositions, which, upon use, provide enhanced anti-bacterial efficacy, enhanced anti-plaque efficacy and/or anti-gingivitis efficacy, in combination with good foaming properties when applied to the oral cavity and/or enhanced uptake of an anti-bacterial component by the tooth surface. Embodiments of the present invention are directed to these ends.

### SUMMARY

The present invention concerns oral care composition comprising an orally acceptable vehicle, a basic amino acid in free or salt form, calcium carbonate, a source of zinc ions comprising at least one of zinc citrate, zinc lactate, zinc gluconate or zinc oxide or any mixture of any two or more thereof, and at least one surfactant selected from of a nonionic block copolymer surfactant and a betaine zwitterionic surfactant or a mixture thereof, wherein the oral care composition does not comprise any anionic surfactant. Further embodiments of the invention are set forth in the dependent claims.

Moreover, the present inventions concerns the use in an oral care compositions comprising an orally acceptable vehicle, a basic amino acid in free or salt form and calcium carbonate, of the combination of a source of zinc ions comprising at least one of zinc citrate, zinc lactate, zinc gluconate or zinc oxide or any mixture of any two or more thereof and a surfactant system selected from at least one of a nonionic block copolymer surfactant and a betaine zwitterionic surfactant or a mixture thereof, for enhancing the uptake of zinc ions by the surface of a mammalian tooth by applying the composition to the surface of a mammalian tooth or for increasing the foaming of the oral care composition in an oral cavity when applying the composition to the surface of a mammalian tooth in the oral cavity, wherein the oral care composition does not comprise any anionic surfactant.

In some embodiments, the nonionic block polycopolymeres surfactant comprises a poloxamer. Optionally, the poloxamer nonionic surfactant is present in an amount of from 0.25 to 2 wt%, further optionally from 0.5 to 1.5 wt%, still further optionally about 1 wt%, based on the weight of the oral care composition.

Optionally, the poloxamer nonionic surfactant has a polyoxypropylene molecular mass of from 3000 to 5000g/mol and a polyoxyethylene content of from 60 to 80 mol%. Further optionally, the poloxamer nonionic surfactant comprises poloxamer 407.

Optionally, the betaine zwitterionic surfactant is present in an amount of from 0.25 to 2 wt%, further optionally from 0.5 to 1.5 wt%, still further optionally about 1 wt%, based on the weight of the oral care composition.

Optionally, the betaine zwitterionic surfactant comprises a C8 - C16 amidopropyl betaine. Further optionally, the betaine zwitterionic surfactant comprises cocamidopropyl betaine.

Optionally, the particles of precipitated calcium carbonate are present in an amount of from 10 to 50 wt%, further optionally from 25 to 40 wt%, based on the weight of the oral care composition.

Optionally, the particles of precipitated calcium carbonate have an average particle size of no greater than a dentin tubule of a mammalian tooth. Typically, the particles of precipitated calcium carbonate have an average particle size of from 1 to 5 microns.

Optionally, the particles of precipitated calcium carbonate comprises a mixture of first [larger] particles having a particle size range of from 1 to 7 microns and second [smaller] particles having a particle size range of from 0.5 to 6 microns. Typically, the first particles are present in an amount of from 5 to 20 wt% based on the weight of the oral care composition and the second particles are present in an amount of from 5 to 40 wt% based on the weight of the oral care composition more typically the first particles are present in an amount of from 5 to 15 wt% based on the weight of the oral care composition and the second particles are present in an amount of from 20 to 30 wt% based on the weight of the oral care composition.

Optionally, the basic amino acid in free or salt form comprises arginine bicarbonate.

Optionally, the basic amino acid in free or salt form is present in an amount of from 5 to 15 wt%, further optionally from 7 to 12 wt%, based on the weight of the oral care composition.

The source of zinc ions comprises at least one of zinc citrate, zinc lactate, zinc gluconate or zinc oxide, or any mixture of any two or more thereof.

Optionally, the source of zinc ions is present in an amount of from 0.5 to 3 wt%, further optionally from 1 to 2 wt%, based on the weight of the oral care composition.

Optionally, the source of zinc ions comprises a mixture of zinc citrate and zinc oxide. Further optionally, zinc citrate is present in an amount of from 0.25 to 0.75 wt% based on the weight of the oral care composition and zinc oxide is present in an amount of from 0.75 to 1.25 wt% based on the weight of the oral care composition.

Optionally, the orally acceptable vehicle comprises sorbitol which is present in an amount of from 12 to 25 wt%, further optionally from 15 to 20 wt%, based on the weight of the oral care composition.

Optionally, the oral care composition does not comprise any sodium lauryl sulfate.

Optionally, the composition is formulated into a dentifrice in the form of a paste or gel.

Optionally, the composition is formulated into a form adapted to be applied undiluted within the oral cavity directly to the surface of a mammalian tooth and to be retained within the cavity on the surface for a period of at least 1 hour for treating or preventing hypersensitivity of the tooth.

Optionally, the oral care composition is for enhancing the uptake of zinc ions by the surface of a mammalian tooth in a method comprising applying the oral care composition to the tooth surface.

Optionally, the oral care composition is for increasing the foaming of the oral care composition in an oral cavity in a method comprising applying the composition to the surface of a mammalian tooth in the oral cavity.

The invention further relates to the use of the oral care compositions of the invention in a method of reducing dental sensitivity comprising applying an oral care composition of the invention to the surface of a mammalian tooth.

The invention further relates to the use of the oral care compositions of the invention in a method of occluding a dentin tubule within the surface of a mammalian tooth comprising applying to the tooth surface a composition according to the invention.

The invention further relates to the use of the oral care compositions of the invention in a method of enhancing the uptake of zinc ions by the surface of a mammalian tooth comprising applying to the tooth surface a composition according to the invention.

The compositions may contain additional therapeutic and non-therapeutic components, and may also be utilized in the practice of various methods, all of which are included within the scope of the invention. The composition and methods within the scope of the invention may be useful in, for example, reducing or eliminating tooth sensitivity of a mammal, improving/maintaining systemic health, and/or occluding dentin tubules.

The present invention is at least partly predicated on the finding by the present inventors that a modified surfactant system in combination with a source of metal ions as an anti-bacterial component can provide enhanced foaming and/or metal ion uptake by the tooth surface of an oral care composition comprising a basic amino acid in free or salt form and particles of precipitated calcium carbonate for treating or relieving hypersensitivity.

In some embodiments, the composition can be formulated into a dentifrice in the form of a paste or gel, in particular a dentifrice suitable for use when brushing the teeth, causing foam formation in the oral cavity.

In other embodiments, the composition can be formulated for use as a "leave-on" oral care composition which can be applied to the tooth surface and can be left within the oral cavity for an extended period of time without causing fluoride damage to the teeth or irritation from the surfactant system. Further, the compositions can be formulated to have a viscosity and rheology so that they can be dispensed directly in an undiluted form onto a tooth surface using an applicator in order to provide relief against dental hypersensitivity, such as a dispenser extruding a narrow cross-section extrudate of the composition.

### DETAILED DESCRIPTION

It should be understood that the detailed description and specific examples, while indicating embodiments of the invention, are intended for purposes of illustration only and are not intended to limit the scope of the invention.

As referred to herein, all compositional percentages are by weight of the total composition, unless otherwise specified.

As stated above, the system for relieving dentin hypersensitivity includes a basic amino acid in free or salt form and particles of precipitated calcium carbonate. Optionally, the particles of precipitated calcium carbonate are present in an amount of from 10 to 50 wt%, or from 25 to 40 wt%, based on the weight of the oral care composition.

Optionally, the particles of precipitated calcium carbonate have an average particle size of no greater than a dentin tubule of a mammalian tooth. Typically, the particles of precipitated calcium carbonate have an average particle size of from 1 to 5 microns.

Optionally, the particles of precipitated calcium carbonate comprises a mixture of first [larger] particles having a particle size range of from 1 to 7 microns and second particles having a particle size range of from 0.5 to 6 microns.

Typically, the first particles are present in an amount of from 5 to 20 wt% based on the weight of the oral care composition and the second particles are present in an amount of from 5 to 40 wt% based on the weight of the oral care composition. More typically, the first particles are present in an amount of from 5 to 15 wt% based on the weight of the oral care composition and the second particles are present in an amount of from 20 to 30 wt% based on the weight of the oral care composition.

Optionally, the basic amino acid in free or salt form comprises arginine. In some embodiments, the basic amino acid in free or salt form comprises arginine bicarbonate.

Optionally, the basic amino acid in free or salt form is present in an amount of from 5 to 15 wt%, or from 7 to 12 wt%, based on the weight of the oral care composition.

Optionally, the oral care composition further comprises silica particles which have an average particle size of no greater than a dentin tubule of a mammalian tooth. Such silica particles may be included for relieving dentin hypersensitivity. Typically, the silica particles have an average particle size of from 1 to 5 microns. Optionally, the silica particles are present in an amount of from 2 to 10 wt%, or from 3 to 6% by weight, based on the total weight of the oral care composition.

In some embodiments, the oral care composition comprises a source of zinc ions as an anti-plaque/anti-gingivitis component. Zinc ions have an anti-bacterial efficacy when used in the oral cavity, which in turn can act to reduce plaque and/or gingivitis.

In some embodiments, the source of zinc ions comprises at least one of zinc citrate, zinc lactate, zinc gluconate or zinc oxide, or any mixture of any two or more thereof.

The source of zinc ions may be present in an amount of from 0.5 to 3 wt%, further optionally from 1 to 2 wt%, based on the weight of the oral care composition.

In some embodiments, the source of zinc ions comprises a mixture of zinc citrate and zinc oxide. In those embodiments, zinc citrate may be present in an amount of from 0.25 to 0.75 wt% based on the weight of the oral care composition and zinc oxide may be present in an amount of from 0.75 to 1.25 wt% based on the weight of the oral care composition.

The compositions may contain additional therapeutic and non-therapeutic components, and may also be utilized in the practice of various methods, all of which are included within the scope of the invention. The composition and methods within the scope of the invention may be useful in, for example, reducing or eliminating tooth sensitivity of a mammal, improving/maintaining systemic health, and/or occluding dentin tubules.

Additional anti-bacterial agents may be incorporated in the oral care compositions of the invention, in addition to the source of zinc ions. Common antibacterial agents used in oral care include triclosan, chlorhexidine, cetyl pyridinium chloride, and other quaternary amines. These agents, when present, are incorporated in the oral care composition in effective amounts that do not substantially adversely affect the desired properties and characteristics of the composition.

Surfactants are used in the oral care composition of the invention to provide foaming, taste, flavor, texture and mouthfeel properties to the compositions, in particular to render the compositions more cosmetically acceptable. In particular embodiments, the surfactants used in the composition of the invention are employed to provide such properties which are substantially cosmetically equivalent to dentifrice compositions incorporating sodium lauryl sulfate. The surfactant components are each a detersive material that imparts to the composition detersive and foaming properties.

In some embodiments, the oral care composition comprises a surfactant system selected from at least one of a poloxamer nonionic surfactant and a betaine zwitterionic surfactant or a mixture thereof. In preferred embodiments, the oral care composition does not comprise any sodium lauryl sulfate, and more preferably the oral care composition does not comprise any anionic surfactant.

In some embodiments, the poloxamer nonionic surfactant is present in an amount of from 0.25 to 2 wt%, further optionally from 0.5 to 1.5 wt%, still further optionally about 1 wt%, based on the weight of the oral care composition.

Optionally, the poloxamer nonionic surfactant has a polyoxypropylene molecular mass of from 3000 to 5000g/mol and a polyoxyethylene content of from 60 to 80 mol%. Typically, the poloxamer nonionic surfactant comprises poloxamer 407, which is available in commerce as Pluronic F-127 (Pluronic is a trade mark of BASF Corporation).

In some embodiments, the betaine zwitterionic surfactant is present in an amount of from 0.25 to 2 wt%, further optionally from 0.5 to 1.5 wt%, still further optionally about 1 wt%, based on the weight of the oral care composition.

Typically, the betaine zwitterionic surfactant comprises a C8 - C16 amidopropyl betaine. More typically, the betaine zwitterionic surfactant comprises cocamidopropyl betaine.

Other surfactants, which may be anionic, cationic, zwitterionic, amphoteric or nonionic, and are known for use in oral care compositions, may optionally be present in the composition.

In some embodiments, the oral compositions of the invention also include a polymeric adherent material to assist in the retention of the calcium carbonate particles, and, if present, the silica particles, within the dentin tubules under salivary flow and during exposure to acidic foods and beverages.

The polymeric adherent material may be any known or to be developed in the art that attaches to the surface of a mammalian tooth and/or to the heterogeneous biofilm which also may be present on a tooth's surface. Attachment may occur by any means, such as ionic interaction, van der Waals forces, hydrophobic-hydrophilic interactions, etc. The adherent material may be, for example, any homopolymers or copolymers (hereinafter referred to collectively as a "polymers") that adhere to the surface of a tooth. Such polymers may include cellulose polymers, for example one or more hydroxyalkyl cellulose polymers, such as hydroxypropylmethyl cellulose (HPMC), hydroxyethylpropyl cellulose (HEPC), hydroxybutylmethyl cellulose (HBMC), carboxymethyl cellulose (CMC).

In some embodiments, the polymeric adherent material comprises at least one cellulose material, for example sodium carboxymethyl cellulose.

The polymers may alternatively or additionally include poly (ethylene oxide) polymers (such as POLYOX from Dow Chemical), linear PVP and cross-linked PVP, PEG/PPG copolymers (such as BASF Pluracare L1220), ethylene oxide (EO) - propylene oxide (PO) block copolymers (such as polymers sold under the trade mark Pluronic available from BASF Corporation), ester gum, shellac, pressure sensitive silicone adhesives (such as BioPSA from Dow-Corning), methacrylates, or mixtures thereof. In an embodiment, a copolymer comprises (PVM/MA). In an embodiment, a copolymer comprises poly (methylvinylether/maleic anhydride). In another embodiment, a copolymer comprises poly (methylvinylether/maleic acid). In another embodiment, a copolymer comprises poly (methylvinylether/maleic acid) half esters. In another embodiment, a copolymer comprises poly (methylvinylether/maleic acid) mixed salts.

Polymers of any molecular weight may be used, including, for example molecular weights of 50,000 to 500,000, 500,000 to 2,500,000 or 2,500,000 to 10,000,000 (calculated by either number average or weight average).

In some embodiments, the oral care compositions of the invention may also contain a source of fluoride ions or fluorine-providing component, as anticaries agent in amount sufficient to supply about 25 ppm to 5,000 ppm of fluoride ions and include inorganic fluoride salts, such as soluble alkali metal salts. For example, preferred fluoride sources are sodium fluoride, potassium fluoride, sodium fluorosilicate, ammonium fluorosilicate, sodium monofluorophosphate as well as tin fluorides, such as stannous fluoride and stannous chloride. Sodium monofluorophosphate is preferred.

However, in some preferred embodiments, the dentifrice does not comprise any fluoride compound or source of fluorine ions. Such embodiments are desired by some consumers who wish to avoid fluorine as an active component in their dentifrice composition. Furthermore, in leave-on compositions no fluoride compound or source of fluorine ions is typically present, to avoid overexposure of the teeth and gums to fluoride ions.

In addition to fluoride compounds, there may also be included antitartar agents such as pyrophosphate salts including dialkali or tetraalkali metal pyrophosphate salts such as Na₄P₂O₇, K₄P₂O₇, Na₂K₂P₂O₇, Na₂H₂P₂O₇ and K₂H₂P₂O₇ sodium tripolyphosphate, long chain polyphosphates such as sodium hexametaphosphate and cyclic phosphates such as sodium trimetaphosphate. These antitartar agents may be included in the oral care composition at a concentration of 1 to 5 wt%.

In some embodiments, the oral care compositions of the invention comprise an orally acceptable vehicle. As used herein, an "orally acceptable vehicle" refers to a material or combination of materials that are safe for use in the compositions of the present invention, commensurate with a reasonable benefit/risk ratio.

The expressions "vehicle" or "aqueous vehicle" as used throughout this description denote any safe and effective materials for use herein. Such materials include, for example, thickening agents, humectants, ionic active ingredients, buffering agents, anticalculus agents, abrasive polishing materials, peroxide sources, alkali metal bicarbonate salts, titanium dioxide, coloring agents, flavor systems, sweetening agents, antimicrobial agents, herbal agents, desensitizing agents, stain reducing agents, and mixtures thereof.

Orally-acceptable vehicles used to prepare the oral care composition of the invention may include a water-phase containing at least one humectant.

The humectant concentration typically totals about 5 to about 75% by weight of the composition. Optionally, the orally acceptable vehicle comprises at least one humectant which is present in an amount of from 15 to 60 wt% based on the weight of the composition, further optionally from 20 to 35 wt% based on the weight of the composition.

Optionally, the at least one humectant comprises a mixture of humectants, such as any mixture of sorbitol, glycerin and/or xylitol.

In some embodiments, the orally acceptable vehicle comprises sorbitol which is present in an amount of from 12 to 25 wt%, further optionally from 15 to 20 wt%, based on the weight of the oral care composition. Reference herein to sorbitol refers to the material typically commercially available as a 70 wt% aqueous solution. In other words, when the orally acceptable vehicle comprises from 12 to 25 wt% sorbitol, this means the active sorbitol concentration is from 8.4 to 17.5 wt%, each amount being based on the weight of the oral care composition.

In other embodiments, the orally acceptable vehicle comprises glycerin which is present in an amount of from 15 to 35 wt% based on the weight of the oral care composition, further optionally from 20 to 30 wt% based on the weight of the oral care composition.

Water is present typically in amount of at least about 10 wt%, and generally about 25 to 70 wt% of the dentifrice composition. Water employed in the preparation of commercially suitable oral compositions should preferably be deionized and free of organic impurities. These amounts of water include the free water which is added plus that which is introduced with other materials such as with sorbitol.

Optionally, the orally acceptable vehicle further comprises at least one cellulose polymer selected from one or more of hydroxypropylmethyl cellulose (HPMC), hydroxyethylpropyl cellulose (HEPC), hydroxybutylmethyl cellulose (HBMC), and carboxymethyl cellulose (CMC). Typically, the at least one cellulose polymer is present in an amount of from 0.5 to 2.5 wt% based on the weight of the oral care composition, more typically from 0.7 to 1.5 wt% based on the weight of the oral care composition.

Commercially-available polymers may be used in the present invention. It is understood that over time, the exact size, weight and/or composition of a commercially-available polymer may change. Based on the disclosure set forth herein, the skilled artisan will understand how to determine whether such polymers are useful in the invention.

In some embodiments, the oral care composition may in particular be a dentifrice composition which may be a toothpaste or a gel and in particular is formulated fir use in brushing the teeth. When formulated as a dentifrice, the composition may comprise a fluorine or fluoride compound for providing anti-cavity efficacy.

In other embodiments, the composition is formulated as a "leave-on" composition which can be applied undiluted and left in the oral cavity for an extended period of time. Such a composition does not include any components or additives which would cause damage or irritation to the oral cavity. Preferably, the "leave-on" composition is formulated into a form adapted to be applied undiluted within the oral cavity directly to the surface of a mammalian tooth and to be retained within the cavity on the surface for a period of at least 1 hour for treating or preventing hypersensitivity of the tooth.

The composition according to the invention may also comprise one or more further agents typically selected from an anti-plaque agent, a whitening agent, antibacterial agent, cleaning agent, a flavouring agent, a sweetening agent, adhesion agents, foam modulators, abrasives, pH modifying agents, humectants, mouth feel agents, colorants, abrasive, tartar control (anticalculus) agent, saliva stimulating agent, nutrient and combinations thereof.

Preferably, specific materials and compositions to be used in this invention are, accordingly, pharmaceutically- or cosmetically-acceptable, clinically effective, and/or clinically efficacious. As used herein, such a "pharmaceutically acceptable" or "cosmetically acceptable", "clinically effective", and/or "clinically efficacious" component is one that is suitable for use with humans and/or animals and is provided in an appropriate amount (a clinically efficacious amount) to provide the desired therapeutic, prophylactic, sensory, decorative, or cosmetic benefit without undue adverse side effects (such as toxicity, irritation, and allergic response) commensurate with a reasonable benefit/risk ratio.

The oral care compositions described herein may be formulated into any delivery form that permits contact of the amino acid, the calcium carbonate particles and the zinc ions to the tooth surface. For example, the compositions may be formulated into a mouth rinse, a paste, a gel, a lozenge (dissolvable or chewable), a spray, a gum, and a film (wholly or partially dissolvable, or indissoluble). The composition may contain any conventional excipients or carriers, although these will vary depending on the dosage form or means of dosage selected.

Excipients or carriers can include, for example, humectants, colorants, flavorants, glycerin, sorbitol, xylitol, water or other solvents, gum bases, thickening agents, surfactants, carrageenan (rich moss), xanthan gum and sodium carboxymethyl cellulose, starch, polyvinyl pyrrolidone, hydroxyethyl propyl cellulose, hydroxybutyl methyl cellulose, hydroxypropyl methyl cellulose, and hydroxyethyl cellulose and amorphous silicas.

Suitable thickeners include naturally occurring polymers such as carrageenan, xanthan gum, polyglycols of varying molecular weights sold under the tradename Polyox, and polyvinylpyrrolidone. Compatible inorganic thickeners include amorphous silica compounds which function as thickening agents and include colloidal silicas compounds available under the trade designation Cab-o-sil manufactured by Cabot Corporation and distributed by Lenape Chemical, Bound Brook, N.J.; Zeodent 165 from J. M. Huber Chemicals Division, Havre de Grace, Md. 21078; and Sylodent 15, available from Davison Chemical Division of W. R. Grace Corporation, Baltimore, Md. 21203. Other inorganic thickeners include natural and synthetic clays such as hectorite clays, lithium magnesium silicate (laponite) and magnesium aluminum silicate (Veegum).

The thickening agent preferably is present in the oral care composition in amounts of 0.1 to 10 wt%, preferably 3 to 7 wt% based on the weight of the oral care composition.

Thickeners particularly suitable of use in the oral care composition of the invention include natural and synthetic gums and colloids. Optionally, the orally acceptable vehicle comprises at least one gum selected from carrageenan and xanthan gum. Further optionally, the orally acceptable vehicle comprises from 0.1 to 0.3 wt% xanthan gum based on the weight of the oral care composition.

The oral care composition of the invention may also contain a flavoring agent. Flavoring agents that are used in the practice of the invention include essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Of these, the most commonly employed are the oils of peppermint and spearmint.

The flavoring agent may be incorporated in the oral care composition at a concentration of 0.1 to 5 wt% and typically 0.5 to 1.5 wt%.

Sweeteners may also be present in the oral care compositions. Artificial sweeteners include sodium saccharin, sucralose and potassium acesulfame. The one or more sweeteners agent may be incorporated in the oral care composition at a concentration of 0.05 to 2 wt%.

Various other materials may be incorporated in the oral care compositions of this invention, including desensitizers, such as potassium nitrate; whitening agents; preservatives; silicones; coloring agents; and chlorophyll compounds. These additives, when present, are incorporated in the oral care composition in amounts that do not substantially adversely affect the properties and characteristics desired.

The oral care composition of the invention may be prepared by any means known in the art. For example, preparation methods for dentifrices are well known, for example, as described in US-B-3966863; US-B-3980767; US-B-4328205; and US-B-4358437, the contents of which are incorporated herein by reference. In general, any humectant (e.g., glycerin, sorbitol, propylene glycol, and/or polyethylene glycol) is dispersed in water in a conventional mixer under agitation. Into that dispersion are added the thickeners, such as carboxylmethyl cellulose (CMC), carrageenan, or xanthan gum; any anionic polycarboxylate; any salts, such as sodium fluoride anticaries agents; and any sweeteners.

The resultant mixture is agitated until a homogeneous gel phase is formed. Into the gel phase are added any pigments utilized, such as TiO₂, and additionally any acid or base required to adjust the pH of the composition. These ingredients are mixed until a homogeneous phase is obtained.

The mixture is then transferred to a high speed/vacuum mixer, wherein the surfactant ingredients are added to the mixture. The calcium carbonate particles and any silica particles utilized are added subsequently. Any water insoluble agents, such as triclosan, are solubilized in the flavor oils to be included in the dentifrice, and that solution is added to the mixture, which is then mixed at high speed in the range from 5 to 30 minutes, under a vacuum of 20 to 50 mm of Hg. The resultant product is a homogeneous, semi-solid, extrudable paste or gel product.

The oral care composition according to the present invention may be administered to or applied to a human or other animal subject. The composition is suitable for administration or application to the oral cavity of a human or animal subject.

In an embodiment, the amino acid and calcium carbonate particle-containing composition may be applied to the tooth via conventional brushing techniques (e.g., use of a toothbrush). In another embodiment, such a composition may be applied to the tooth via a method other than conventional brushing techniques. Other methods of application, particularly for "leave-on" compositions, include manual application (e.g., applying a composition to a tooth using one or more fingers, rubbing onto the tooth surface, rubbing in a circular motion, etc....), or application using any known dental appliance or applicator, for example which extrudes the composition onto the teeth. It will be understood, based on the disclosure set forth herein, that any method of smearing a composition onto a tooth, optionally using varying degrees of physical pressure, is encompassed by the invention.

Desensitization of a tooth according to the invention may be measured by any technique set forth herein, or any technique known to the skilled artisan.

Application of the composition to the tooth surface results in the introduction of the composition into one or more dentin tubules. The composition is applied to the teeth by any method set forth herein or known in the art.

The invention also includes within its scope several related methods. For example, the invention includes within its scope methods of reducing and methods of occluding a dentin tubule of a mammalian tooth, methods of protecting dentin from acid-mediated degradation, and methods of reducing dental sensitivity.

Each of these methods includes the steps of applying any of the compositions described above to the tooth surface. Application may be carried out by any method, so long as the adherent material and the particles are placed in contact with the tooth surface. Application may be accomplished by brushing, flossing, prophylaxis, irrigating, wiping, rinsing (lavage of oral cavity), foam/gel and in-tray application, masticating, spraying, painting, etc., or applied by film or strip.

Dental sensitivity may be reduced according to a method of the invention by applying a composition of the invention to a tooth surface. A composition may be applied using a traditional method, as described in detail elsewhere herein, or by any appliance or applicator, whether or not typically associated with dental use. In an embodiment, one or more human fingers is used to apply a dental sensitivity-reducing composition to one or more teeth. A finger can be used to smear the composition on the surface of a tooth, or to otherwise apply the composition to the surface of a tooth.

The application may be at least once a day, although up to five times per day may be preferred, and may be carried out over a duration of time, e.g., one week, up to one year, up to three years or for a lifetime.

Various embodiments now will be described with reference to the following nonlimiting examples.

### EXAMPLES

### Comparative Examples 1 and 2

Dentifrice compositions having the formula of Table 1 are prepared.

The compositions of Comparative Examples 1 and 2 both included arginine bicarbonate and precipitated calcium carbonate which is known to provide efficacy against dental hypersensitivity. However, the composition of Comparative Example 2 differed from that of Comparative Example 1 by the addition of 1 wt% zinc oxide (ZnO) as an anti-bacterial active to provide anti-plaque and anti-gingivitis efficacy.

As shown in Table 1 (below), both of these compositions comprised an anionic surfactant, in particular sodium lauryl sulfate (SLS).

**Table 1**

| **Ingredient** | **Comp. Ex. 1** | **Comp. Ex. 2** |
|---|---|---|
| Sorbitol (70 wt% aq. soln.) | 23 | 23 |
| Sodium CMC | 0.72 | 0.72 |
| Sodium bicarbonate | 0.5 | 0.5 |
| N-silicate | 0.8 | 0.8 |
| Titanium dioxide | 0.5 | 0.5 |
| Precipitated calcium carbonate - Light | 10 | 10 |
| Precipitated calcium carbonate - High Absorption | 25 | 25 |
| Arginine bicarbonate (70 wt% aq. solution) | 13.86 | 13.86 |
| Xanthan gum | 0.135 | 0.135 |
| Sodium saccharin | 0.3 | 0.3 |
| Sodium monofluorophosphate | 1.1 | 1.1 |
| Flavor | 1.1 | 1.1 |
| Sodium lauryl sulfate | 1.5 | 1.5 |
| Zinc oxide | -- | 1 |
| Water | QS | QS |

When used to brush the teeth, it is important to the consumer that the dentifrice forms a stable foam in the oral cavity. To evaluate the foam properties of the dentifrice compositions of Comparative Examples 1 and 2, the compositions were mixed with water and agitated to form a foam. A given amount of composition was agitated with a given amount of water. The amount of foam formed in a tube, in an *in vitro* test, was quantified after 2 minutes. The results are shown in Table 2 (below).

**Table 2**

| | Foam Volume (Comparative Units) |
|---|---|
| Comparative Example 1 - SLS, arginine and calcium carbonate | 284 |
| Comparative Example 2 - SLS, arginine and calcium carbonate, 1 wt% ZnO | 28 |
| Comparative Example 3 - SLS and calcium carbonate, 1 wt% ZnO, no arginine | 283 |
| Comparative Example 4 - SLS and calcium carbonate, 0.5 wt% ZnO, 2 wt% zinc citrate | 37 |
| Comparative Example 5 - arginine and calcium carbonate, 1 wt% ZnO, polysorbate 20 | 0 |
| Example 1 - arginine, calcium carbonate, 1 wt% ZnO, 1 wt% poloxamer 407 | 78 |
| Example 2 - arginine, calcium carbonate, 1 wt% ZnO, 2 wt% poloxamer 407 | 161 |
| Example 3 - arginine, calcium carbonate, 1 wt % ZnO, 3.4 wt% cocamidopropyl betaine (30% active) | 153 |
| Example 4 - arginine, calcium carbonate, 1 wt% ZnO, 0.5 wt% zinc citrate, 1 wt% poloxamer 407, 3.4 wt% cocamidopropyl betaine (30% active) | 161 |

It may be seen that the SLS-containing composition of Comparative Example 1 exhibited good foam properties, but that the addition of ZnO to the composition significantly deteriorated the foam properties.

The invention at least partly aimed to overcome the foam reduction effect of introducing a source of zinc ions into an oral care composition including a basic amino acid in free or salt form and particles of precipitated calcium carbonate.

### Comparative Examples 3 and 4

Dentifrice compositions having the formula of Table 3 are prepared.

The compositions of Comparative Example 3 and 4 both also included precipitated calcium carbonate and SLS. However, the composition of Comparative Example 3 did not include arginine bicarbonate whereas the composition of Comparative Example 4 included the same amount of arginine bicarbonate as Comparative Example 1. Comparative Examples 3 and 4 each included, as sources of zinc ions, 0.5 wt% zinc oxide (ZnO) and 2.0 wt% zinc citrate.

**Table 3**

| **Ingredient** | **Comp. Ex. 3** | **Comp. Ex. 4** |
|---|---|---|
| Sorbitol (70 wt% aq. soln.) | 29.93 | 23 |
| Sodium CMC | 0.72 | 0.72 |
| Sodium bicarbonate | 0.5 | 0.5 |
| N-silicate | 0.8 | 0.8 |
| Titanium dioxide | 0.5 | 0.5 |
| Precipitated calcium carbonate - Light | 10 | 10 |
| Precipitated calcium carbonate - High Absorption | 25 | 25 |
| Arginine bicarbonate (70 wt% aq. solution) | -- | 13.86 |
| Xanthan gum | 0.135 | 0.135 |
| Sodium saccharin | 0.3 | 0.3 |
| Sodium monofluorophosphate | 1.1 | 1.1 |
| Flavor | 1.1 | 1.1 |
| Sodium lauryl sulfate | 1.5 | 1.5 |
| Zinc oxide | 0.5 | 0.5 |
| Zinc citrate trihydrate | 2 | 2 |
| Benzyl alcohol | 0.3 | -- |
| Water | QS | QS |

To evaluate the foam properties of the dentifrice compositions of Comparative Examples 3 and 4, the compositions were tested for foam volume as for Comparative Examples 1 and 2. The results are shown in Table 2 (above).

It may be seem that when arginine is absent from the calcium carbonate-containing composition as in Comparative Example 3, the combination of SLS and the source of zinc ions does not significantly reduce the foam volume properties as compared to Comparative Example 1 which contained arginine but no zinc ions.

A comparison of Comparative Examples 3 and 4 shows that when arginine is added to the SLS and calcium carbonate-containing composition as in Comparative Example 3,which also contains the source of zinc ions, the foam volume properties are not significantly reduced. This comparative data shows that there is some combined effect resulting from the arginine presence which affects the foam volume properties in the presence of SLS and zinc ions.

### Example 1

The oral care composition of Example 1 was modified as compared to Comparative Example 1 by including a surfactant system which did not include any anionic surfactant or SLS, but instead included a nonionic surfactant, in particular 1 wt% poloxamer 407 (Pluronic F-127), in combination with the arginine and calcium carbonate components and ZnO.

The composition is shown in Table 4.

The oral care composition of Example 1 was also subjected to the same *in vitro* foam test and the results are shown in Table 2 (above).

**Table 4**

| **Ingredient** | **Ex. 1** | **Ex. 2** | **Ex. 3** | **Ex. 4** |
|---|---|---|---|---|
| Sorbitol (70 wt% aq. soln.) | 23 | 23 | 23 | 23 |
| Sodium CMC | 0.72 | 0.72 | 0.72 | 0.72 |
| Sodium bicarbonate | 0.5 | 0.5 | 0.5 | 0.5 |
| N-silicate | 0.8 | 0.8 | 0.8 | 0.8 |
| Titanium dioxide | 0.5 | 0.5 | 0.5 | 0.5 |
| Precipitated calcium carbonate - Light | 10 | 10 | 10 | 10 |
| Precipitated calcium carbonate - High Absorption | 25 | 25 | 25 | 25 |
| Arginine bicarbonate (70 wt% aq. solution) | 13.86 | 13.86 | 13.86 | 13.86 |
| Xanthan gum | 0.135 | 0.135 | 0.135 | 0.135 |
| Sodium saccharin | 0.3 | 0.3 | 0.3 | 0.3 |
| Sodium monofluorophosphate | 1.1 | 1.1 | 1.1 | 1.1 |
| Flavor | 1.1 | 1.1 | 1.1 | 1.1 |
| Poloxamer 407 | 1 | 2 | -- | 1 |
| Cocamidopropyl betaine (30 wt% aq. soln.) | -- | -- | 3.34 | 3.34 |
| Zinc oxide | 1 | 1 | 1 | 1 |
| Zinc citrate trihydrate | -- | -- | -- | 0.5 |
| Water | QS | QS | QS | QS |

Table 2 shows that by employing a poloxamer surfactant as compared to the SLS surfactant in Comparative Example 2 the foam quality is increased.

Also, foam volume is increased as compared to Comparative Example 4 by changing the surfactant from SLS.

### Example 2

The oral care composition of Example 2 was modified as compared to Example 1 by including 2 wt% poloxamer 407 (Pluronic F-127), in combination with the arginine and calcium carbonate components and ZnO. The composition is shown in Table 4.

The oral care composition of Example 2 was also subjected to the same *in vitro* foam test and the results are shown in Table 2 (above). Table 2 shows that increasing the poloxamer 407 content increased the foam volume, and providing 2 wt% poloxamer 407 provided a foam volume not significantly lower than for the composition of Comparative Example 1 which did not comprise any zinc ions.

Also, foam volume was increased as compared to Comparative Example 4 by changing the surfactant from SLS.

### Example 3

The oral care composition of Example 3 was modified as compared to Example 1 by including 3.4 wt% of cocamidopropyl betaine (30 wt% active component) instead of the poloxamer 407 (Pluronic F-127), in combination with the arginine and calcium carbonate components and ZnO. The composition is shown in Table 4.

The oral care composition of Example 3 was also subjected to the same *in vitro* foam test and the results are shown in Table 2 (above). Table 2 shows that the cocamidopropyl betaine provided a similar foam volume as compared to the poloxamer 407 of Example 2.

Also, foam volume was increased as compared to Comparative Example 4 by changing the surfactant from SLS.

### Example 4

The oral care composition of Example 3 was modified as compared to Example 3 by including a combined surfactant system of not only 3.4 wt% of cocamidopropyl betaine (30 wt% active component)but also 1 wt% poloxamer 407 (Pluronic F-127), in combination with the arginine and calcium carbonate components. Furthermore, the zinc ions were provided not only by 1 wt% ZnO but also by 0.5 wt% zinc citrate. The composition is shown in Table 4.

The oral care composition of Example 4 was also subjected to the same *in vitro* foam test and the results are shown in Table 2 (above). Table 2 shows that the combined surfactant system of cocamidopropyl betaine and poloxamer 407 provided a similar foam volume as compared to Example 2 even though there was a higher zinc content because of the addition of 0.5 wt% zinc citrate to the 1.0 wt% zinc oxide content.

Also, foam volume was increased as compared to Comparative Example 4 by changing the surfactant from SLS.

The composition of Example 4 which included the combined surfactant system unexpectedly provided the best foam volume properties for a given zinc ion content when used in the oral care composition comprising arginine and calcium carbonate.

### Comparative Example 5

The oral care composition of Comparative Example 5 was modified as compared to Example 1 by employing a polysorbate 20 nonionic surfactant rather than the poloxamer surfactant. The polysorbate 20 nonionic surfactant was present in an amount of 1 wt%, and in combination with the same arginine and calcium carbonate components. Furthermore, the zinc ions were similarly provided by 1 wt% ZnO. The composition is shown in Table 5 (below).

**Table 5**

| **Ingredient** | **Comp. Ex. 5** |
|---|---|
| Sorbitol (70 wt% aq. soln.) | 23 |
| Sodium CMC | 0.72 |
| Sodium bicarbonate | 0.5 |
| N-silicate | 0.8 |
| Titanium dioxide | 0.5 |
| Precipitated calcium carbonate - Light | 10 |
| Precipitated calcium carbonate - High Absorption | 25 |
| Arginine bicarbonate (70 wt% aq. solution) | 13.86 |
| Xanthan gum | 0.135 |
| Sodium saccharin | 0.3 |
| Sodium monofluorophosphate | 1.1 |
| Flavor | 1.1 |
| Polysorbate 20 | 1 |
| Zinc oxide | 1 |
| Water | QS |

The oral care composition of Comparative Example 5 was also subjected to the same *in vitro* foam test and the results are shown in Table 2 (above). Table 2 shows that the polysorbate 20 nonionic surfactant provided significantly worse foam volume as compared to Example 1. This shows that the non-ionic surfactant systems of the present invention provide enhanced foam volume as compared to other nonionic surfactant systems.

### Example 5

The oral care composition of Example 5 included a surfactant system which did not include any anionic surfactant or SLS, but instead included a surfactant system in accordance with the invention, in combination with the arginine and calcium carbonate components and a source of zinc ions.

The composition is shown in Table 6 (below).

**Table 6**

| **Ingredient** | **Ex. 5** | **Comp. Ex. 6** | **Comp. Ex. 7** |
|---|---|---|---|
| Sorbitol (70 wt% aq. soln.) | 23 | 23 | 23 |
| Sodium CMC | 0.72 | 0.72 | 0.8 |
| Sodium bicarbonate | 0.5 | 0.5 | 0.5 |
| N-silicate | 0.8 | 0.8 | 0.8 |
| Titanium dioxide | 0.5 | 0.5 | 0.5 |
| Precipitated calcium carbonate - Light | 10 | 10 | 10 |
| Precipitated calcium carbonate - High Absorption | 25 | 25 | 25 |
| Arginine bicarbonate (70 wt% aq. solution) | 13.86 | 13.86 | -- |
| Xanthan gum | 0.135 | 0.135 | 0.135 |
| Sodium saccharin | 0.3 | 0.3 | 0.3 |
| Sodium monofluorophosphate | 1.1 | 1.1 | 1.1 |
| Flavor | 1.1 | 1.1 | 1.1 |
| Polysorbate 20 | 0.50 | -- | -- |
| Cocamidopropyl betaine (30 wt% aq. soln.) | 3.34 | -- | -- |
| Sodium Lauryl Sulfate | -- | 1.5 | 1.5 |
| Zinc oxide | 1 | 1 | 1 |
| Water | QS | QS | QS |

The oral care composition of Example 5 was subjected to an *in vitro* test to determine the take up of zinc on a saliva-coated hydroxyapatite disk. The results are shown in Table 7 (below).

**Table 7**

| | **Zinc Uptake Mµg/disk** |
|---|---|
| Example 5 | 304 |
| Comparative Example 6 | 235 |
| Comparative Example 7 | 28 |

### Comparative Examples 6 and 7

The oral care composition of Comparative Example 6 was modified as compared to Example 5 by employing SLS anionic surfactant rather than the surfactant system of the invention.

The oral care composition of Comparative Example 7 was modified as compared to Example 5 by not including arginine.

The oral care compositions of Comparative Examples 6 and 7 were subjected to the same *in vitro* test to determine the take up of zinc on a saliva-coated hydroxyapatite disk. The results are also shown in Table 7.

Table 7 shows that by employing a surfactant system in accordance with the invention as compared to the SLS surfactant in Comparative Example 6 the zinc uptake was increased.

Also, Table 7 shows that when no arginine is present in the composition, as in Comparative Example 7, the zinc uptake is minimal.

Accordingly, when used in a composition comprising arginine, zinc ions and calcium carbonate, the surfactant system used in the invention increased zinc ion uptake as compared to the use of SLS and compared to a composition not comprising arginine.

## Claims

1. An oral care composition comprising an orally acceptable vehicle, a basic amino acid in free or salt form, calcium carbonate, a source of zinc ions comprising at least one of zinc citrate, zinc lactate, zinc gluconate or zinc oxide or any mixture of any two or more thereof, and at least one surfactant selected from of a nonionic block copolymer surfactant and a betaine zwitterionic surfactant or a mixture thereof, wherein the oral care composition does not comprise any anionic surfactant.

2. The oral care composition according to claim 1 wherein the calcium carbonate is present in an amount of from 10 to 50 wt% based on the weight of the oral care composition, optionally in an amount of from 25 to 40 wt% based on the weight of the oral care composition.

3. The oral care composition according to any one of claims 1 to 2 wherein the calcium carbonate has an average particle size of no greater than a dentin tubule of a mammalian tooth,
and/or wherein the calcium carbonate has an average particle size of from 1 to 5 microns.

4. The oral care composition according to any of claims 1 to 3 wherein the calcium carbonate comprises a mixture of first particles having a particle size range of from 1 to 7 microns and second particles having a particle size range of from 0.5 to 6 microns,
optionally wherein the first particles are present in an amount of from 5 to 20 wt% based on the weight of the oral care composition and the second particles are present in an amount of from 5 to 40 wt% based on the weight of the oral care composition,
further optionally wherein the first particles are present in an amount of from 5 to 15 wt% based on the weight of the oral care composition and the second particles are present in an amount of from 20 to 30 wt% based on the weight of the oral care composition.

5. The oral care composition according to any one of claims 1 to 4 wherein the basic amino acid in free or salt form comprises arginine bicarbonate,
and/or wherein the basic amino acid in free or salt form is present in an amount of from 5 to 15 wt% based on the weight of the oral care composition, optionally in an amount of from 7 to 12 wt% based on the weight of the oral care composition.

6. The oral care composition according to any one of claims 1 to 5 wherein the zinc ion source is present in an amount of from 0.5 to 3 wt% based on the weight of the oral care composition, optionally in an amount of from 1 to 2 wt% based on the weight of the oral care composition.

7. The oral care composition according to claim 6 wherein the zinc ion source comprises a mixture of zinc citrate and zinc oxide,
optionally wherein zinc citrate is present in an amount of from 0.25 to 0.75 wt% based on the weight of the oral care composition and zinc oxide is present in an amount of from 0.75 to 1.25 wt% based on the weight of the oral care composition.

8. The oral care composition according to any one of claims 1 to 6, wherein the basic amino acid in free or salt form comprises arginine bicarbonate, the zinc ion source comprises zinc oxide, and wherein the composition comprises a nonionic block copolymer surfactant comprising poloxamer 407,
or wherein the basic amino acid in free or salt form comprises arginine bicarbonate, the zinc ion source comprises zinc oxide, and wherein the composition comprises a betaine zwitterionic surfactant comprising cocamidopropyl betaine.

9. The oral care composition according to any one of claims 1 to 7, wherein the basic amino acid in free or salt form comprises arginine bicarbonate, the zinc ion source comprises a mixture of zinc citrate and zinc oxide, and wherein the composition comprises a nonionic block copolymer surfactant comprising poloxamer 407 and a betaine zwitterionic surfactant comprising cocamidopropyl betaine.

10. The oral care composition according to any one of claims 1 to 9 wherein the orally acceptable vehicle comprises sorbitol which is present in an amount of from 12 to 30 wt% based on the weight of the oral care composition, optionally in an amount of from 15 to 25 wt% based on the weight of the oral care composition.

11. The oral care composition according to any one of claims 1 to 10 wherein the composition is formulated into a dentifrice in the form of a paste or gel,
and/or wherein the composition is formulated into a form adapted to be applied undiluted within the oral cavity directly to the surface of a mammalian tooth and to be retained within the cavity on the surface for a period of at least 1 hour for treating or preventing hypersensitivity of the tooth.

12. The oral care composition according to any one of claims 1 to 11 for enhancing the uptake of zinc ions by the surface of a mammalian tooth or for increasing the foaming of the oral care composition in an oral cavity in a method comprising applying the oral care composition to the tooth surface.

13. The oral care composition according to any one of claims 1 to 12, wherein the composition further comprises stannous fluoride in an amount sufficient to supply 25 ppm to 5,000 ppm of fluoride ions.

14. Use, in an oral care composition comprising an orally acceptable vehicle, a basic amino acid in free or salt form and calcium carbonate, of the combination of a source of zinc ions comprising at least one of zinc citrate, zinc lactate, zinc gluconate or zinc oxide or any mixture of any two or more thereof and a surfactant system selected from at least one of a nonionic block copolymer surfactant and a betaine zwitterionic surfactant or a mixture thereof, for enhancing the uptake of zinc ions by the surface of a mammalian tooth by applying the composition to the surface of a mammalian tooth or for increasing the foaming of the oral care composition in an oral cavity when applying the composition to the surface of a mammalian tooth in the oral cavity,
wherein the oral care composition does not comprise any anionic surfactant.

15. The oral care composition according to any one of claims 1 to 13 or the use according to claim 14, wherein the nonionic block copolymer is present in an amount of from 0.25 to 2 wt% based on the weight of the oral care composition, optionally in an amount of from 0.5 to 1.5 wt% based on the weight of the oral care composition, further optionally in an amount of about 1 wt% based on the weight of the oral care composition.

16. The oral care composition according to any one of claims 1 to 13 or claim 15, or the use according to any one of claims 14 to 15, wherein the nonionic block copolymer surfactant comprises a poloxamer, optionally wherein the poloxamer nonionic surfactant comprises poloxamer 407.

17. The oral care composition according to any one of claims 1 to 13 or 15 to 16, or the use according to any one of claims 14 to 16, wherein the nonionic block copolymer surfactant has a polyoxypropylene molecular mass of from 3000 to 5000g/mol and a polyoxyethylene content of from 60 to 80 mol%.

18. The oral care composition according to any one of claims 1 to 13 or 15 to 17, or the use according to any one of claims 14 to 17, wherein the betaine zwitterionic surfactant is present in an amount of from 0.25 to 2 wt% based on the weight of the oral care composition, optionally in an amount of from 0.5 to 1.5 wt% based on the weight of the oral care composition, further optionally in an amount of about 1 wt% based on the weight of the oral care composition.

19. The oral care composition according to any one of claims 1 to 13 or 15 to 18, or the use according to any one of claims 14 to 18, wherein the betaine zwitterionic surfactant comprises a C₈-C₁₆ amidopropyl betaine, optionally wherein the betaine zwitterionic surfactant comprises cocamidopropyl betaine.

## Patentansprüche

1. Mundpflegezusammensetzung umfassend einen oralannehmbaren Träger, eine basische Aminosäure in freier oder Salz- Form, Kalziumkarbonat, eine Quelle von Zinkionen umfassend mindestens eines aus Zinkzitrat, Zinklaktat, Zinkglukonat oder Zinkoxid oder einer beliebigen Mischung von beliebigen zwei oder mehreren davon, und mindestens ein Tensid ausgewählt aus einem nicht-ionischen Blockcopolymer Tensid und eine Betain Zwitterion Tensid oder eine Mischung davon, wobei die Mundpflegezusammensetzung nicht irgendein anionisches Tensid umfasst.

2. Mundpflegezusammensetzung gemäß Anspruch 1, worin das Kalziumkarbonat in einer Menge von 10 bis 50 Gew.% vorliegt, bezogen auf das Gewicht der Mundpflegezusammensetzung, gegebenenfalls in einer Menge von 25 bis 40 Gew.%, bezogen auf das Gewicht der Mundpflegezusammensetzung.

3. Mundpflegezusammensetzung gemäß irgendeinem der Ansprüche 1 bis 2, worin das Kalziumkarbonat eine durchschnittliche Teilchengröße von nicht mehr als ein Dentin Tubulus eines Säugetierzahns hat, und/oder worin das Kalziumkarbonat eine durchschnittliche Teilchengröße von 1 bis 5 Mikrometern hat.

4. Mundpflegezusammensetzung gemäß irgendeinem der Ansprüche 1 bis 3, worin das Kalziumkarbonat eine Mischung aus ersten Teilchen mit einem Teilchengrößenbereich von 1 bis 7 Mikrometern und zweiten Teilchen mit einem Teilchengrößenbereich von 0,5 bis 6 Mikrometern umfasst, wobei gegebenenfalls die ersten Teilchen in einer Menge von 5 bis 20 Gew.%, bezogen auf das Gewicht der Mundpflegezusammensetzung, vorliegen und die zweiten Teilchen in einer Menge von 5 bis 40 Gew.%, bezogen auf das Gewicht der Mundpflegezusammensetzung, vorliegen,
weiterhin gegebenenfalls worin die ersten Teilchen in einer Menge von 5 bis 15 Gew.%, bezogen auf das Gewicht der Mundpflegezusammensetzung, vorliegen und die zweiten Teilchen liegen in einer Menge von 20 bis 30 Gew.% vor, bezogen auf das Gewicht der Mundpflegezusammensetzung.

5. Mundpflegezusammensetzung gemäß irgendeinem der Ansprüche 1 bis 4, worin die basische Aminosäure in freier oder Salz- Form Argininbikarbonat umfasst, und/oder worin die basische Aminosäure in freier oder Salz- Form in einer Menge von 5 bis 15 Gew.% vorliegt, bezogen auf das Gewicht der Mundpflegezusammensetzung, gegebenenfalls in einer Menge von 7 bis 12 Gew.%, bezogen auf das Gewicht der Mundpflegezusammensetzung.

6. Mundpflegezusammensetzung gemäß irgendeinem der Ansprüche 1 bis 5, worin die Zinkionenquelle in einer Menge von 0,5 bis 3 Gew.%, bezogen auf das Gewicht der Mundpflegezusammensetzung, vorliegt, gegebenenfalls in einer Menge von 1 bis 2 Gew.%, bezogen auf das Gewicht der Mundpflegezusammensetzung.

7. Mundpflegezusammensetzung gemäß Anspruch 6, worin die Zinkionenquelle eine Mischung von Zinkzitrat und Zinkoxid umfasst, gegebenenfalls worin das Zinkzitrat in einer Menge von 0,25 bis 0,75 Gew.% vorliegt, bezogen auf das Gewicht der Mundpflegezusammensetzung, und Zinkoxid liegt in einer Menge von 0,75 bis 1,25 Gew.%, bezogen auf das Gewicht der Mundpflegezusammensetzung, vor.

8. Mundpflegezusammensetzung gemäß irgendeinem der Ansprüche 1 bis 6, worin die basische Aminosäure in freier oder Salz- Form Arginibikarbonat umfasst, die Zinkionenquelle umfasst Zinkoxid und wobei die Zusammensetzung ein nicht-ionisches Blockcopolymer Tensid umfassend Poloxamer 407 umfasst;
oder worin die basische Aminosäure in freier oder Salz- Form Argininbikarbonat umfasst, die Zinkionenquelle umfasst Zinkoxid, und wobei die Zusammensetzung ein Betain-Zwitterion-Tensid umfassend Cocamidopropyl Betain umfasst.

9. Mundpflegezusammensetzung gemäß irgendeinem der Ansprüche gemäß 1 bis 7, worin die basische Aminosäure in freier oder Salz- Form Argininbikarbonat umfasst, die Zinkionenquelle umfasst eine Mischung aus Zinkzitrat und Zinkoxid, und wobei die Zusammensetzung ein nicht-ionisches Blockcopolymer Tensid umfassend Poloxamer 407 und ein Betain-Zwitterion-Tensid umfassend Cocamidopropyl Betain umfasst.

10. Mundpflegezusammensetzung gemäß irgendeinem der Ansprüche 1 bis 9, worin der oral annehmbare Träger Sorbitol umfasst, das in einer Menge von 12 bis 30 Gew.%, bezogen auf das Gewicht der Mundpflegezusammensetzung, vorliegt, gegebenenfalls in einer Menge von 15 bis 25 Gew.%, bezogen auf das Gewicht der Mundpflegezusammensetzung.

11. Mundpflegezusammensetzung gemäß irgendeinem der Ansprüche 1 bis 10, worin die Zusammensetzung zu einer Zahnpasta in Form einer Paste oder eines Gels formuliert ist, und/oder worin die Zusammensetzung in eine Form formuliert ist, die angepasst wurde innerhalb der Mundhöhle auf die Oberfläche eines Säugetierzahns unverdünnt aufgebracht zu werden und innerhalb der Mundhöhle auf der Oberfläche über einen Zeitraum von mindestens einer Stunde zurückgehalten zu werden, um Hypersensitivität des Zahns zu behandeln oder diese zu verhindern.

12. Mundpflegzusammensetzung gemäß irgendeinem der Ansprüche 1 bis 11 zur Steigerung der Aufnahme von Zinkionen durch die Oberfläche eines Säugetierzahns oder zur Steigerung der Schäumung der Mundpflegezusammensetzung in einer Mundhöhle in einem Verfahren, das das Auftragen der Mundpflegezusammensetzung auf die Zahnoberfläche umfasst.

13. Mundpflegezusammensetzung gemäß irgendeinem der Ansprüche 1 bis 12, worin die Zusammensetzung weiterhin umfasst Zinnflourid in einer Menge ausreichend um 25 ppm bis 5000 ppm Flouridionen bereitzustellen.

14. Verwendung, in einer Mundpflegezusammensetzung umfassend einen oral annehmbaren Träger, eine basische Aminosäure in freier oder Salz- Form und Kalziumkarbonat, von einer Kombination einer Zinkionenquelle umfassend mindestens eines von Zinkzitrat, Zinklaktat, Zinkglukonat oder Zinkoxid oder eine beliebige Mischung von beliebigen zwei oder mehreren davon und einem Tensidsystem ausgewählt aus mindestens einem nicht-ionischen Blockcopolymer Tensid und einem Betain-Zwitterion-Tensid oder einer Mischung davon, zur Steigerung der Aufnahme von Zinkionen durch die Oberfläche eines Säugetierzahns durch Auftragen der Zusammensetzung auf die Oberfläche eines Säugetierzahns oder zur Steigerung des Schäumens der Mundpflegezusammensetzung in einer Mundhöhle, wenn die Zusammensetzung auf die Oberfläche eines Säugetierzahns in der Mundhöhle aufgebracht wird, wobei die Mundpflegezusammensetzung nicht ein beliebiges anionisches Tensid umfasst.

15. Mundpflegezusammensetzung gemäß irgendeinem der Ansprüche 1 bis 13 oder die Verwendung nach Anspruch 14, wobei das nicht-ionische Blockcopolymer Tensid in einer Menge von 0,25 bis 2 Gew.%, bezogen auf das Gewicht der Mundpflegezusammensetzung, vorliegt, gegebenenfalls in einer Menge von 0,5 bis 1,5 Gew.%, bezogen auf das Gewicht der Mundpflegezusammensetzung, und weiterhin gegebenenfalls in einer Menge von etwa 1 Gew.%, bezogen auf das Gewicht der Mundpflegezusammensetzung.

16. Mundpflegezusammensetzung gemäß irgendeinem der Ansprüche 1 bis 13 oder Anspruch 15, oder die Verwendung gemäß irgendeinem der Ansprüche 14 bis 15, wobei das nicht-ionische Blockcopolymer Tensid ein Poloxamer umfasst, gegebenenfalls wobei das Poloxamer nicht-ionische Tensid Poloxamer 407 umfasst.

17. Mundpflegezusammensetzung gemäß irgendeinem der Ansprüche 1 bis 13 oder 15 bis 16, oder die Verwendung gemäß irgendeinem der Ansprüche 14 bis 16, wobei das nicht-ionische Blockcopolymer Tensid eine Polyoxypropylenmolekularmasse von 3000 bis 5000g/Mol hat und einen Polyoxyethylengehalt von 60 bis 80 Mol%.

18. Mundpflegezusammensetzung gemäß irgendeinem der Ansprüche 1 bis 13 oder 15 bis 17, oder die Verwendung gemäß irgendeinem der Ansprüche 14 bis 17, wobei das Betain-Zwitterion-Tensid in einer Menge von 0,25 bis 2 Gew.% vorliegt, bezogen auf das Gewicht der Mundpflegezusammensetzung, gegebenenfalls in einer Menge von 0,5 bis 1,5 Gew.%, bezogen auf das Gewicht der Mundpflegezusammensetzung, weiterhin gegebenenfalls in einer Menge von etwa 1 Gew.%, bezogen auf das Gewicht der Mundpflegezusammensetzung.

19. Mundpflegezusammensetzung gemäß irgendeinem der Ansprüche 1 bis 13 oder 15 bis 18, oder die Verwendung gemäß irgendeinem der Ansprüche 14 bis 18, wobei das Betain-Zwitterion-Tensid ein (C₈-C₁₆-Amidopropyl-Betain umfasst, gegebenenfalls, worin das Betain-Zwitterion-Tensid Cocamidopropyl Betain umfasst.

## Revendications

1. Composition de soin buccal comprenant un véhicule acceptable par voie orale, un acide aminé basique sous forme libre ou sous forme de sel, du carbonate de calcium, une source d'ions zinc comprenant au moins un élément parmi le citrate de zinc, le lactate de zinc, le gluconate de zinc ou l'oxyde de zinc ou n'importe quel mélange de deux ou plus d'entre eux, et au moins un tensioactif choisi parmi un tensioactif copolymère séquencé non ionique et un tensioactif zwitterionique de type bétaïne ou un mélange de ceux-ci, dans laquelle la composition de soin buccal ne comprend pas de tensioactif anionique.

2. Composition de soin buccal selon la revendication 1, dans laquelle le carbonate de calcium est présent en une quantité de 10 à 50 % en poids par rapport au poids de la composition de soin buccal, éventuellement en une quantité de 25 à 40 % en poids par rapport au poids de la composition de soin buccal.

3. Composition de soin buccal selon l'une quelconque des revendications 1 à 2, dans laquelle le carbonate de calcium a une taille moyenne de particule non supérieure à un tubule dentinaire d'une dent de mammifère,
et/ou dans laquelle le carbonate de calcium a une taille moyenne de particule comprise entre 1 et 5 microns.

4. Composition de soin buccal selon l'une quelconque des revendications 1 à 3, dans laquelle le carbonate de calcium comprend un mélange de premières particules ayant une plage de taille de particule comprise entre 1 et 7 microns et des secondes particules ayant une plage de taille de particule comprise entre 0,5 et 6 microns,
éventuellement dans laquelle les premières particules sont présentes en une quantité de 5 à 20 % en poids par rapport au poids de la composition de soin buccal et les secondes particules sont présentes en une quantité de 5 à 40 % en poids par rapport au poids de la composition de soin buccal,
éventuellement, en outre, dans laquelle les premières particules sont présentes en une quantité de 5 à 15 % en poids par rapport au poids de la composition de soin buccal et les secondes particules sont présentes en une quantité de 20 à 30 % en poids par rapport au poids de la composition de soin buccal.

5. Composition de soin buccal selon l'une quelconque des revendications 1 à 4, dans laquelle l'acide aminé basique sous forme libre ou sous forme de sel comprend le bicarbonate d'arginine,
et/ou dans laquelle l'acide aminé basique sous forme libre ou sous forme de sel est présent dans une quantité de 5 à 15 % en poids par rapport au poids de la composition de soin buccal, éventuellement en une quantité de 7 à 12 % en poids par rapport au poids de la composition de soin buccal.

6. Composition de soin buccal selon l'une quelconque des revendications 1 à 5, dans laquelle la source d'ions zinc est présente en une quantité de 0,5 à 3 % en poids par rapport au poids de la composition de soin buccal, éventuellement en une quantité de 1 à 2 % en poids par rapport au poids de la composition de soin buccal.

7. Composition de soin buccal selon la revendication 6, dans laquelle la source d'ions zinc comprend un mélange de citrate de zinc et d'oxyde de zinc,
éventuellement dans laquelle le citrate de zinc étant présent en une quantité de 0,25 à 0,75 % en poids par rapport au poids de la composition de soin buccal et l'oxyde de zinc est présent en une quantité de 0,75 à 1,25 % en poids par rapport au poids de la composition de soin buccal.

8. Composition de soin buccal selon l'une quelconque des revendications 1 à 6, dans laquelle l'acide aminé basique sous forme libre ou sous forme de sel comprend du bicarbonate d'arginine, la source d'ions zinc comprend de l'oxyde de zinc, et dans laquelle la composition comprend un tensioactif copolymère séquencé non ionique comprenant du poloxamère 407,
ou dans laquelle l'acide aminé basique sous forme libre ou sous forme de sel comprend du bicarbonate d'arginine, la source d'ions zinc comprend de l'oxyde de zinc, et dans laquelle la composition comprend un tensioactif zwitterionique de type bétaïne comprenant de la cocamidopropyl bétaïne.

9. Composition de soin buccal selon l'une quelconque des revendications 1 à 7, dans laquelle l'acide aminé basique sous forme libre ou sous forme de sel comprend du bicarbonate d'arginine, la source d'ions zinc comprend un mélange de citrate de zinc et d'oxyde de zinc, et dans laquelle la composition comprend un tensioactif copolymère séquencé non ionique comprenant du poloxamère 407 et un tensioactif zwitterionique de type bétaïne comprenant de la cocamidopropyl bétaïne.

10. Composition de soin buccal selon l'une quelconque des revendications 1 à 9, dans laquelle le véhicule acceptable par voie orale comprend du sorbitol qui est présent en une quantité de 12 à 30 % en poids par rapport au poids de la composition de soin buccal, éventuellement en une quantité de de 15 à 25 % en poids par rapport au poids de la composition de soin buccal.

11. Composition de soin buccal selon l'une quelconque des revendications 1 à 10, dans laquelle la composition est formulée en un dentifrice sous la forme d'une pâte ou d'un gel,
et/ou dans laquelle la composition est formulée sous une forme adaptée pour être appliquée non diluée dans la cavité buccale directement à la surface d'une dent de mammifère et pour être retenue dans la cavité sur la surface pendant une période d'au moins 1 heure afin de traiter ou de prévenir une hypersensibilité de la dent.

12. Composition de soin buccal selon l'une quelconque des revendications 1 à 11 pour améliorer l'absorption d'ions zinc par la surface d'une dent de mammifère ou pour augmenter le moussage de la composition de soin buccal dans une cavité buccale dans un procédé comprenant l'application de la composition de soin buccal à la surface de la dent.

13. Composition de soin buccal selon l'une quelconque des revendications 1 à 12, dans laquelle la composition comprend en outre du fluorure stanneux en une quantité suffisante pour fournir 25 ppm à 5 000 ppm d'ions fluorure.

14. Utilisation, dans une composition de soin buccal comprenant un véhicule acceptable par voie orale, un acide aminé sous forme libre ou sous forme de sel et du carbonate de calcium, de la combinaison d'une source d'ions zinc comprenant au moins un élément parmi le citrate de zinc, le lactate de zinc, le gluconate de zinc ou l'oxyde de zinc ou n'importe quel mélange de deux ou plus d'entre eux et d'un système tensioactif choisi parmi un tensioactif copolymère séquencé non ionique et/ou un tensioactif zwitterionique de type bétaïne ou un mélange de ceux-ci, pour améliorer l'absorption d'ions zinc par la surface d'une dent de mammifère par application de la composition à la surface d'une dent de mammifère ou pour augmenter le moussage de la composition de soin buccal dans une cavité buccale lors de l'application de la composition à la surface d'une dent de mammifère dans la cavité buccale,
dans laquelle la composition de soin buccal ne comprend pas de tensioactif anionique.

15. Composition de soin buccal selon l'une quelconque des revendications 1 à 13 ou l'utilisation selon la revendication 14, dans laquelle le copolymère séquencé non ionique est présent en une quantité de 0,25 à 2 % en poids par rapport au poids de la composition de soin buccal, éventuellement en une quantité de 0,5 à 1,5 % en poids par rapport au poids de la composition de soin buccal, éventuellement, en outre, en une quantité d'environ 1 % en poids par rapport au poids de la composition de soin buccal.

16. Composition de soin buccal selon l'une quelconque des revendications 1 à 13 ou la revendication 15, ou l'utilisation selon l'une quelconque des revendications 14 à 15, dans laquelle le tensioactif copolymère séquencé non ionique comprend un poloxamère, éventuellement dans laquelle le tensioactif poloxamère nonionique comprend le poloxamère 407.

17. Composition de soin buccal selon l'une quelconque des revendications 1 à 13 ou 15 à 16, ou l'utilisation selon l'une quelconque des revendications 14 à 16, dans laquelle le tensioactif copolymère séquencé non ionique a une masse moléculaire de polyoxypropylène de 3 000 à 5 000 g/mole et une teneur en polyoxyéthylène de 60 à 80 % en moles.

18. Composition de soin buccal selon l'une quelconque des revendications 1 à 13 ou 15 à 17, ou l'utilisation selon l'une quelconque des revendications 14 à 17, dans laquelle le tensioactif zwitterionique de type bétaïne est présent en une quantité de 0,25 à 2 % en poids par rapport au poids de la composition de soin buccal, éventuellement en une quantité de 0,5 à 1,5 % en poids par rapport au poids de la composition de soin buccal, éventuellement, en outre, en une quantité d'environ 1 % en poids par rapport au poids de la composition de soin buccal.

19. Composition de soin buccal selon l'une quelconque des revendications 1 à 13 ou 15 à 18, ou l'utilisation selon l'une quelconque des revendications 14 à 18, dans laquelle le tensioactif zwitterionique de type bétaïne comprend une amidopropyl bétaïne en C₈-C₁₆, éventuellement dans laquelle le tensioactif zwitterionique de type bétaïne comprend de la cocamidopropyl bétaïne.
